(19) Europäisches Patentamt
European Patent Office
Office européen des brevets

(11) Veröffentlichungsnummer : **0 314 007 B1**

(12) **EUROPÄISCHE PATENTSCHRIFT**

(45) Veröffentlichungstag der Patentschrift : **21.08.91 Patentblatt 91/34**

(51) Int. Cl.[5] : **C07C 37/20**, C07C 39/17, C07D 493/04

(21) Anmeldenummer : **88117549.1**

(22) Anmeldetag : **21.10.88**

# (54) Verfahren zur Herstellung von Bisphenolaromaten.

Die Akte enthält technische Angaben, die nach dem Eingang der Anmeldung eingereicht wurden und die nicht in dieser Patentschrift enthalten sind.

(30) Priorität : **30.10.87 DE 3736814**

(43) Veröffentlichungstag der Anmeldung : **03.05.89 Patentblatt 89/18**

(45) Bekanntmachung des Hinweises auf die Patenterteilung : **21.08.91 Patentblatt 91/34**

(84) Benannte Vertragsstaaten : **AT DE FR GB IT NL**

(56) Entgegenhaltungen :
**EP-A- 0 065 060**
**EP-A- 0 180 133**
**US-A- 4 049 421**

(73) Patentinhaber : **Röhm GmbH**
**Kirschenallee Postfach 4242**
**W-6100 Darmstadt 1 (DE)**

(72) Erfinder : **Knebel, Joachim, Dr.**
**Dieselstrasse 20**
**W-6100 Darmstadt (DE)**
Erfinder : **Kerscher, Volker, Dr.**
**Waldstrasse 58**
**W-6107 Reinheim (DE)**
Erfinder : **Ude, Werner, Dr.**
**Birngartenweg 115**
**W-6100 Darmstadt-Arheilgen (DE)**

**EP 0 314 007 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

### Gebiet der Erfindung

Die Erfindung betrifft ein Verfahren zur Herstellung von Bisphenolaromaten durch Umsetzung von Phenolen mit aromatischen Ketonen, wie z.B. die Herstellung von 9,9-Bis-(4-hydroxyphenyl)fluoren durch Umsetzung von Fluorenon mit Phenol, in Gegenwart saurer Kondensationsmittel.

### Stand der Technik

Zur Herstellung oben genannter Verbindung, die als Zwischenprodukt, insbesondere zur Herstellung von Polykondensationsprodukten Interesse findet, sind mehrere Verfahren beschrieben. Bei diesen bekannten Verfahren wird die Synthese der Verbindung durch Umsetzung von Fluorenon mit Phenol unter Abspaltung von Wasser in Gegenwart von sauren Kondensationsmitteln durchgeführt.

Die Herstellung von 9,9-Bis-(4-hydroxyphenyl)fluoren läßt sich, wie aus der US-PS 4049721 und aus der europäischen Patentschrift 0065060 hervorgeht, mit Chlorwasserstoff, der gasförmig in das Reaktionsgemisch eingeleitet wird, als saurem Kondensationskatalysator durchführen. Als effektive Cokatalysatoren werden dem Chlorwasserstoff nach der oben genannten US-PS, Beispiel 1, β-Mercaptopropionsäure und nach der oben genannten europäischen Patentschrift zwei-, drei- oder vierwertige Metallchloride hinzugegeben.

Ein großer Nachteil beim Arbeiten mit Chlorwasserstoff oder konzentrierter Salzsäure ist die hohe Korrosivität, die diese Agentien bei großtechnischen Herstellungen von 9,9-Bis-(4-hydroxyphenyl)fluoren in den zu verwendenden Metallapparaturen verursachen. Korrosiv wie Chlorwasserstoff oder konzentrierte Salzsäure wirken auch die als Cokatalysatoren bekannten Metallhalogenide. Die Korrosivität der HCl-haltigen Reaktionsgemische wird dadurch verstärkt, daß bei der Aufarbeitung noch Wasser zugesetzt wird. Weiter sind die dann erhaltenen, verdünnten, HCl-haltigen wäßrigen Lösungen schwierig aufarbeitbar.

In der DE-OS 3439484 (entspricht US 4675458) wird ein Verfahren zur Herstellung von 9,9-Bis-(4-hydroxyphenyl)fluoren beschrieben, bei dem als saures Kondensationsmittel Schwefelsäure mit über 70% Schwefelsäuregehalt eingesetzt wird. Dieses Verfahren überwindet das Arbeiten mit den korrosiven HCl-haltigen Reaktionsmischungen bzw. Waschwassern. Die Aufarbeitung der schwefelsäurehaltigen Reaktionsgemische wird zweckmäßigerweise durch deren Verdünnung mit Wasser durchgeführt, wobei verdünnte Schwefelsäuren, die noch weitere Stoffe, insbesondere überschüssiges Phenol enthalten, anfallen. Diese Lösungen müssen entsorgt werden.

Aus Rec. Trav. Chim. 87,599 (1968) ist die Umsetzung von Benzil, einem Diaryldiketon mit Resorcin in Gegenwart von konzentrierter Schwefelsäure als Kondensationsmittel bekannt, wobei 3,8-Dihydroxy-5a, 10b-diphenylcumarano-2',3',2,3-cumaran, ein aromatisches Bisphenol, in 30,4 %-iger Ausbeute erhalten wurde.

Dieses Bisphenol hat die Formel (I) :

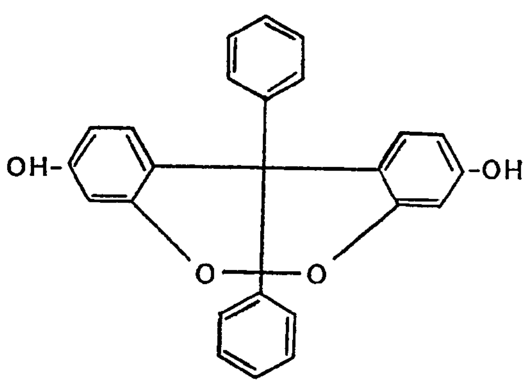

(I)

Für die Herstellung von Bis-(4-hydroxyphenyl)alkanen durch Kondensation von Phenolen mit aliphatischen oder cycloaliphatischen Carbonylverbindungen werden mit Vorteil wasserfreie, unlösliche, Sulfonsäuregruppen enthaltende Kationenaustauscher eingesetzt, wie dies beispielsweise in der DE-OS 2811182, in der DE-PS 2722683 und der EP-A 0023325 beschrieben ist. Dadurch entfallen Entsorgungen verdünnter wäßriger Säurelösungen, und die Verfahrensprodukte fallen in reinerer Form an als beim Arbeiten mit den bisher üblichen sauren, aber wasserlöslichen Kondensationsmitteln.

Aufgabe und Lösung

Für die Umsetzung aromatischer Ketone mit Phenolen zu Bisphenolaromaten war eine Verfahrensweise zu finden, die es gestattet, den sauren Kondensationskatalysator als echten Katalysator einsetzbar zu halten, und somit die ständige Entsorgung wäßriger Katalysatorsäure entbehrlich zu machen.

Es wurde gefunden, daß das Diarylketon Fluorenon und das Diaryldiketon Benzil mit Phenolen in Gegenwart von stark sauren Kationenaustauschern als Kondensationskatalysatoren zu Bisphenolaromaten kondensieren. Der erfindungsgemäß zu verwendende saure Katalysator kann nicht mit Wasser herausgelöst werden und steht theoretisch für unendlich viele Umsetzungsschritte zur Verfügung.

Die gefundene Umsetzung ist überraschend, da nach dem oben genannten Stand der Technik die Verwendung stark saurer Kationenaustauscher als Kondensationskatalysatoren auf die Umsetzung aliphatischer und cycloaliphatischer Carbonylverbindungen mit Phenolen, und dies insbesondere auf die technisch wichtige Bisphenol A-Synthese mit Aceton als Carbonylverbindung beschränkt schien, und nach eigenen Versuchen das bekannte Diarylketon Benzophenon und das araliphatische Keton Acetophenon in Gegenwart saurer Kationenaustauscher mit Phenolen nicht reagierten.

Zur weiteren Erhöhung der Reaktionsgeschwindigkeit bei dem erfindungsgemäßen Verfahren sind schwefelorganische Verbindungen als Cokatalysatoren im Reaktionsgemisch vorteilhaft. Diese können dem Reaktandengemisch als freie Verbindungen zugesetzt werden oder können chemisch an das Ionenautauschharz gebunden sein. Als schwefelorganische Verbindungen eignen sich mercaptogruppenhaltige Verbindungen.

Gegenstand der Erfindung ist daher ein Verfahren zur Herstellung der Bisphenolaromaten 9,9-Bis-(4-hydroxyphenyl)fluoren oder 3,8-Dihydroxy-5a, 10b-diphenylcumarano-2′, 3′, 2,3-cumaran durch Umsetzung von Fluorenon mit Phenol oder von Benzil mit Resorcin in Gegenwart saurer Kondensationsmittel als Katalysatoren und gegevenfalls in Gegenwart einer organischen mercaptogruppen-haltigen Verbindung als Cokatalysator, dadurch gekennzeichnet, daß ein unlösliches, stark saures Kationenaustauscherharz als saures Kondensationsmittel eingesetzt wird.

Ausführung der Erfindung

Das aromatische Diketon Benzil reagiert bevorzugt nur an einer Carbonylgruppe im Sinne der Phenol-Keton-Kondensation. Die zweite Carbonylgruppe kann jedoch weitere Reaktionen eingehen, wie dies beispielsweise für das Umsetzungsprodukt von Benzil mit Resorcin aus der Formel (I) ersichtlich ist.

Das erfindungsgemäße Verfahren wird vorzugsweise mit einer stöchiometrisch überschüssigen Menge an Phenol durchgeführt. Die Durchführung mit einer stöchiometrisch überschüssigen Menge an Diarylketon ist aber auch möglich. Ein Molverhältnis von Diarylketon zu Phenol von 1 : 2 bis 1 : 10 und vor allem von 1 : 3 bis 1 : 6 ist bevorzugt.

Als saure Kondensationskatalysatoren zu verwendende Kunstharz-Ionenaustauscher werden vor allem sulfonierte, vernetzte Polystyrole oder entsprechende Styrol-Acrylat-Copolymere, wie sie als Lewatite® oder Amberlite® erhältlich sind, oder Poly(perfluoralkylen)-sulfonsäure, erhältlich unter dem Namen Nafion®, eingesetzt. Neben den für die Protonenkatalyse verantwortlichen sauren Gruppen, wie vor allem den $SO_3H$-Gruppen, können an den Ionenaustauschharzen, entweder direkt an der Matrix oder über die Sulfonsäuregruppen, noch Molekül-Gruppen mit einer SH-Funktion angebracht sein, wodurch die Mercaptan-Cokatalyse ebenfalls vom Ionenaustauschharz her gesteuert wird. In der EP-A 0023325 ist beispielsweise ein mit Divinylbenzol vernetztes Polystyrol beschrieben, das durch partielle Umsetzung von am Polymerisat eingeführten Sulfochloridgruppen mit Cysteamin, SH-Gruppen-haltige Harzbereiche, neben den durch Hydrolyse von Sulfochloridgruppen entstehenden Sulfonsäuregruppen aufweist. (S. auch EP-A 0049411). Zu Ionenaustauscher s. Ullmanns Enzyklopädie der technischen Chemie, 4. Auflage, Band 13, Seiten 279 und 280, insbesondere Seite 313 und Seiten 534 und 535.

Der Mercaptangruppen-haltige Cokatalysator kann aber auch in ungebundener Form als niedermolekulare Verbindung, z.B. als β-Mercaptopropionsäure, bei der Reaktion vorhanden sein.

Das erfindungsgemäße Verfahren kann kontinuierlich oder diskontinuierlich durchgeführt werden. Die kontinuierliche Arbeitsweise ist bevorzugt. Bei dem diskontinuierlichen Verfahren werden die Reaktionsteilnehmer und der Katalysator zur besseren Kontaktierung und damit zur Raum-Zeit-Ausbeute-Verbesserung gut mechanisch gerührt. Bei einer kontinuierlichen Arbeitsweise werden die Austauschharze, die meist in Form kleiner Perlen oder als Partikel unregelmäßiger Gestalt mit 0,5 bis ca. 5 mm Korngröße angewendet werden, beispielsweise als Festbett in einem vertikalen, röhrenförmigen Reaktor angeordnet und vom Reaktionsgemisch durchströmt.

Die Reaktionstemperatur liegt je nach Art der herzustellenden Verbindung zwischen etwa 20 und etwa 150

Grad C. Temperaturen von 20 bis etwa 100 Grad C sind bevorzugt.

Die Umsetzung kann gegebenenfalls auch in Gegenwart eines inerten Lösungsmittels, wie z.B. Toluol, Cyclohexan oder Fluorbenzol, durchgeführt werden, wobei diese während der Umsetzung gegebenenfalls auch als Wasserschleppmittel dienen können.

Der Druck in der Reaktionszone liegt im allgemeinen zwischen 0,1 und 10 bar, insbesondere zwischen 0,5 und 3 bar.

Die eingesetzten Ionenaustauscher haben H-Ionen-Totalkapazitäten von etwa 2,5 bis etwa 6 mval/g Trockensubstanz.

Ist der Mercaptan-Cokatalysator nicht an das Ionenaustauschharz gebunden, so wird er den Reaktanden in einer Menge von 0,003 bis 0,05 Mol pro Mol umzusetzendem Diarylketon zugesetzt. Als Schwefelverbindung wird beispielsweise β-Mercaptopropionsäure eingesetzt.

Nach der Umsetzung wird, gegebenenfalls nach Verdünnung mit einem wassermischbaren Lösungsmittel, vom Ionenaustauscher abfiltriert. Die Reaktionsprodukt-Lösung kann dann noch eingeengt und zur Entfernung von überschüssigem Phenol mehrmals mit Wasser ausgekocht werden. Ein weiteres effektives Verfahren zur Abtrennung von überschüssigem Phenol ist dessen Entfernung durch Wasserdampfdestillation. Das dann erhaltene Produkt kann zur weiteren Reinigung noch umkristallisiert werden.

Beispiel 1

Ein Gemisch aus 22,5 g (0,125 mol) Fluorenon, 58,8 g (0,625 mol) Phenol, 30 g Amberlyst® 16 (Rohm & Haas) und 0,03 ml β-Mercaptopropionsäure wird in einem Witt'schen Topf mit U-formigem Metallrührer unter Argon vorgelegt und bei 100 Grad C gerührt.

Nach 2 Stunden ist das Fluorenon vollständig umgesetzt (DC-Kontrolle : Kieselgel/Aceton-Cyclohexan 1: 2). Nach Abkühlung auf Raumtemperatur werden 110 ml Isopropanol zugesetzt, erhitzt, heiß vom Ionenaustauscher filtriert und zum Filtrat unter Rühren 110 ml Wasser zugegeben. Der abgekühlte Ionentauscher wird dreimal mit je 100 ml Aceton gewaschen und im Vakuum bei 60 Grad C getrocknet. Die Acetonlösungen werden mit dem gleichen Volumen Wasser versetzt und mit der Isopropanol-Lösung vereinigt.

Das ausgefallene Rohprodukt wird abgesaugt, 3 mal mit je 250 ml Wasser ausgekocht und aus 110 ml Isopropanol umkristallisiert.

Ausbeute : 30 g (68,5 % d. Th) 9,9-Bis(hydroxiphenyl)fluoren ;
Fp : 221 Grad C

Beispiel 2

Wie Beispiel 1, jedoch unter Verwendung von 30 g Lewatit® SPC 118 (Bayer). Reaktionsdauer : 6 Stunden
Ausbeute : 32,8 g (74% d.Th.)
Fp : 222-223 Grad C

Beispiel 3

Wie Beispiel 1, jedoch unter Verwendung des gebrauchten Amberlyst® 16 aus Beispiel 1. Reaktionsdauer: 6 Stunden.
Ausbeute : 26,1 g (59,6% d.Th) 9,9-Bis(hydroxiphenyl)fluoren ;
Fp.222-224 Grad C

Beispiel 4

Ein Gemisch aus 20 g Fluorenon (0,11 Mol), 20,7 g Phenol (0,22 Mol), 5 g Amberlite® XE 386 (Rohm & Haas) 0,03 ml β-Mercaptopropionsäure und 100 ml Fluorbenzol wird am Wasserabscheider unter Rückfluß gelöst bis sich kein Wasser mehr abscheidet (1,5 Stunden). Das Produkt fällt während der Reaktion aus. Man gibt 30 ml Isopropanol zur Reaktionsmischung, erhitzt, filtriert heiß und engt das Filtrat bis zur Trockene ein. Das Rohprodukt wird aus Isopropanol umkristallisiert.
Ausbeute : 29 g (75% d.Th) 9,9-Bis(hydroxyphenyl)fluoren ;
Fp : 224 Grad C

Beispiel 5

Wie Beispiel 4 jedoch unter Verwendung von Cyclohexan als Wasserschleppmittel

Ausbeute : 25 g (65% d.Th)
Fp : 224 Grad C

Beispiel 6

Wie Beispiel 5, jedoch unter Verwendung von Toluol als Wasserschleppmittel. Das Produkt bleibt während der Reaktion in Lösung. Man filtriert direkt vom Ionenaustauscher ab, destilliert das Lösungsmittel (Toluol) ab und kristallisiert aus Isopropanol um.
Ausbeute : 23 g (60% d. Th)
Fp : 224 Grad C

Beispiel 7

Zu einer Lösung von 20 g Benzil (95 mmol) und 21 g Resorcin (190 mmol) in 120 ml Toluol gibt man 10 g stark sauren Ionenaustauscher A-16 (Rohm & Haas) und erhitzt so lange unter Rückfluß, bis kein Wasser mehr abgeschieden wird (2 Stunden). Die resultierende Reationsmischung wird nach dem Erkalten filtriert, zur Trockene eingeengt und dann mit Chloroform (1 × 60 ml) ausgekocht und erneut filtriert. Die verbleibende feste Masse kristallisiert man aus Ethanol/$H_2O$ (2 : 1) um.
Ausbeute : 21,0 g (58% d.Th = 55 mmol) 3,8-Dihydroxy-5a, 10b-diphenyl-cumarano-2',3', 2,3-cumaran.
Schmp. : 254 Grad C

## Patentansprüche

1. Verfahren zur Herstellung der Bisphenolaromaten 9,9-Bis-(4-hydroxyphenyl)fluoren oder 3,8-Dihydroxy-5a,10b-diphenylcumarano-2', 3', 2,3-cumaran durch Umsetzung von Fluorenon mit Phenol oder von Benzil mit Rosorcin in Gegenwart saurer Kondensationsmittel als Katalysatoren und gegebenenfalls in Gegenwart einer organischen mercaptogruppenhaltigen Verbindung als Cokatalysator, dadurch gekennzeichnet, daß ein unlösliches, stark saures Kationenaustauscherharz als saures Kondensationsmittel eingesetzt wird.

2. Verfahren nach Anspruch 1, dadurch gekennzeichnet, daß die Umsetzung im Temperaturbereich von 20 bis 150 Grad C durchgeführt wird.

3. Verfahren nach den Ansprüchen 1 und 2, dadurch gekennzeichnet, daß die Umsetzung in Gegenwart von inerten Lösemitteln durchgeführt wird.

4. Verfahren nach den Ansprüchen 1 bis 3, dadurch gekennzeichnet, daß als Cokatalysator β-Mercaptopropionsäure eingesetzt wird.

5. Verfahren nach den Ansprüchen 1 bis 3, dadurch gekennzeichnet, daß das saure kationische Austauschharz selbst freie Mercaptogruppen enthält.

## Claims

1. Process for preparing the bisphenol aromatic compounds 9,9-bis-(4-hydroxyphenyl)fluorine or 3,8-dihydroxy-5a,10b-diphenylcumarano-2',3', 2,3-cumarane by reacting fluorenone with phenol or by reacting benzil with resorcinol in the presence of acidic condensing agents as catalysts and optionally in the presence of an organic compound which contains mercapto groups as cocatalyst, characterised in that an insoluble, strongly acidic cation exchanger resin is used as the acid condensation agent.

2. Process according to claim 1, characterised in that the reaction is carried out at temperatures in the range from 20 to 150°C.

3. Process according to claims 1 and 2, characterised in that the reaction is carried out in the presence of inert solvents.

4. Process according to claims 1 to 3, characterised in that β-mercaptopropionic acid is used as co-catalyst.

5. Process according to claims 1 to 3, characterised in that the acid cationic exchanger resin itself contains free mercapto groups.

## Revendications

1. Procédé de préparation des carbures aromatiques bisphénoliques 9,9-bis-(4-hydroxyphényl)-fluorène

ou 3,8-dihydroxy-5a,10b-diphénylcoumarono-2',3', 2,3-coumaranne par réaction de fluorénone avec le phénol ou de benzile avec la résorcine en présence d'agents de condensation acides en tant que catalyseurs et éventuellement en présence d'un composé organigue contenant des groupements mercapto en tant que co-catalyseur, caractérisé en ce qu'on utilise, comme agent de condensation acide, une résine échangeuse de cations fortement acide insoluble.

2. Procédé selon la revendication 1, caractérisé en ce que la réaction est conduite dans la gamme de température de 20 à 150°C.

3. Procédé selon la revendication 1 ou 2, caractérisé en ce que la réaction est conduite en présence de solvants inertes.

4. Procédé selon l'une quelconque des revendications 1 à 3, caractérisé en ce que de l'acide β-mercaptopropionique est utilisé comme co-catalyseur.

5. Procédé selon l'une quelconque des revendications 1 à 3, caractérisé en ce que la résine échangeuse de cations acide contient elle-même des groupements mercapto libres.